(19) Europäisches Patentamt
European Patent Office
Office européen des brevets

(11) Publication number: 0 077 583
B1

(12) **EUROPEAN PATENT SPECIFICATION**

(45) Date of publication of patent specification: **28.08.85**

(51) Int. Cl.$^4$: **C 09 B 61/00**, A 23 L 1/275

(21) Application number: **82201246.4**

(22) Date of filing: **08.10.82**

(54) **Method for extracting astaxanthin.**

(30) Priority: **20.10.81 IT 2457181**

(43) Date of publication of application: **27.04.83 Bulletin 83/17**

(45) Publication of the grant of the patent: **28.08.85 Bulletin 85/35**

(84) Designated Contracting States: **AT BE CH DE FR GB LI LU NL SE**

(56) References cited:
**FR-A-2 483 227**
**FR-A-2 483 228**
**US-A-3 906 112**

**CHEMICAL ABSTRACTS, vol. 88, no. 13, March 1978, page 384, no. 87897m, Columbus, Ohio, USA**
**JOURNAL OF AGRICULTURAL AND FOOD CHEMISTRY, vol. 29, 1981, pages 505-508, American Chemical Society, Washington D.C., USA**

(73) Proprietor: **ANIC S.p.A.**
**Via Ruggero Settimo, 55**
**I-90139 Palermo (IT)**

(72) Inventor: **Celia, Giovanni**
**Via Moro 13**
**I-20097 S. Donato Milanese (Milan) (IT)**

(74) Representative: **Roggero, Sergio et al**
**Ing. Barzanò & Zanardo Milano S.p.A. Via Borgonuovo 10**
**I-20121 Milano (IT)**

**EP 0 077 583 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The flesh of trout and salmon, when in their natural environment, has a colour which varies from pink to red. This coloration is not due to a component necessarily present in the flesh itself, but is the result of the depositing of carotenoids (mainly astaxanthin) which are present in the crustacea which form the food of the aforementioned fish, namely shrimps and zooplankton.

In recent years, production farms for trout and salmon have increased considerably, but the flesh of these latter is of a different or softer colour than the flesh of the natural fish, which could be a symptom of insufficient or at least different feeding. For this reason, special methods have been developed for returning the flesh coloration of fish to tones very close to natural tones, these methods being based on the use of synthetic canthaxanthin and dried and/or frozen shrimps which are administered with the diet.

The aforesaid substances however have certain drawbacks, including the following:

— canthaxanthin is synthesized chemically, and enters the human alimentary cycle by way of the fish flesh, accompanied by all the problems which arise from this in terms of toxicity; in addition, the colour of the fish flesh is unstable with time;
— shrimp or prawn shells, a waste product deriving from their shelling, decompose too rapidly, are voluminous, and moreover the addition of large quantities thereof to the diet can cause alterations in the fish mineral content in the medium and long term.

The aforesaid problems have suggested the use of astaxanthin, and all research activity is now centred on the production and extraction thereof.

This product is extracted at the present time from crustacean shells using triglyceride oils of animal or vegetable origin (US. 3,906,112) as an alternative to methods which use more or less polar solvents such as acetone, hexane, ethanol, ether and the like, which give rise to a series of disadvantages and drawbacks such as process difficulties and product instability.

Extraction with oil is motivated by the fast that astaxanthin is sufficiently stable in a solution thereof, and also for commercial reasons in that astaxanthin/oil solutions of different proportions have been marketed. However, oil as an extraction medium is not free from drawbacks.

Firstly, the pigment must be extracted at high temperature (90—150°C), which can lead to its partial decomposition if a suitable antioxidant is not added. In addition, the residual oil must be removed from the shells if these are to be subsequently used for producing chitin and/or chitosan.

Finally, when extraction is carried out at a temperature of less than 100°C, it is difficult to remove the water contained in the organic extract due to the fact that it is emulsified.

If the extraction were to be carried out above 100°C, a long period would be necessary for evaporating all the water contained in the shells, leading to considerable complications from the industrial aspect and a high energy consumption.

The applicant has now discovered a method, constituting the subject matter of the present invention, for extracting astaxanthin from crustacean shells which besides resulting in a high yield does not suffer from any of the aforesaid drawbacks.

In carrying out the extraction, the method in question uses a solvent chosen from carboxylic acid esters of the general formula

$$R^1\text{—}\overset{\overset{\displaystyle O}{\|}}{C}\text{—}OR^2$$

in which $R^1$ is hydrogen or an alkyl radical with preferably up to 5 carbon atoms, and $R^2$ is again an alkyl radical which can be the same as or different from $R^1$; particular advantages have been found by using acetic esters.

The extraction can be carried out over the entire temperature range in which the solvent is in its liquid state, but it is preferable to operate between 15 and 25°C in that this temperature range gives high extraction yields without requiring critical operating conditions.

The operation can also be carried out in air, as the astaxanthin does not oxidise due to the relatively mild extractive conditions. In addition, the use of special antioxidants is superfluous.

The weight ratio of dry shells to solvent can vary widely according to the process used (continuous or batch), the apparatus used (type of agitation, type of filtration etc.) and the required final astaxanthin concentration.

Particular advantages have been found by operating at a ratio of between 1:3 and 1:5.

The extraction time is of the order of 4.5 minutes, but goes to completion in a time of 10—20 minutes. The average yield is 10—40% higher than the yield obtained by the usual extraction methods.

It has however been noted that the yield is also related to the time for which the shells have been stored, and the storage temperature.

In general, an increase in storage time leads to a yield reduction because of astaxanthin decomposition.

Example 1

2.13 kg of prawn shells containing 47% of dry matter, preserved for ten days at −25°C and having a size of 3—4 mm (ground with a rotating knife mill) were mixed with ethyl acetate in a weight ratio of 1:5 with respect to the dry matter and kept stirring for about 20 minutes. After this, they were filtered under pressure and the filtrate was collected and evaporated to constant weight. Spectrophotometric analysis was carried out on

the semisolid residue consisting essentially of fatty substances and free astaxanthin and/or its esters for the quantitative determination thereof.

392 mg of astaxanthin were extracted by this procedure.

Example 2

Using an analogous method to that described in Example 1, but under a stream of nitrogen, 368.86 mg of astaxanthin per kilo of dry shells were extracted.

Example 3

Under the same conditions as described in Example 1 but carrying out the extraction at 60°C, 371.4 mg of astaxanthin per kilo of dry shells were extracted.

Example 4

Under the same extraction conditions as described in Example 1 but using methyl acetate as the extraction solvent, 350 mg of astaxanthin per kilo of dry shells were extracted.

**Claims**

1. A method for extracting astaxanthin from crustacean shells consisting of treating the shells with a solvent chosen from carboxylic acid alkyl esters of general formula

$$R^1{-}\overset{O}{\overset{\|}{C}}OR^2$$

in which $R^1$ is hydrogen or an alkyl radical of up to 5 carbon atoms, and $R^2$ is an alkyl radical the same as or different from $R^1$.

2. A method for extracting astaxanthin as claimed in the preceding claim, wherein the ratio of dry shells to solvent varies between 1:3 and 1:5.

3. A method for extracting astaxanthin as claimed in claim 1, wherein the operation is carried out within the temperature range in which the solvent is in the liquid state.

4. A method for extracting astaxanthin as claimed in the preceding claim, wherein the operation is preferably carried out at a temperature of between 15 and 25°C.

**Patentansprüche**

1. Methode zum Extrahieren von Astaxanthin aus Krustazeen-Muscheln, darin bestehend, daß die Muscheln mit einem Lösungsmittel behandelt werden, ausgewählt aus Carbonsäure-alkylestern der allgemeinen Formel

$$R^1{-}\overset{O}{\overset{\|}{C}}OR^2$$

worin $R^1$ Wasserstoff oder ein Alkylrest mit bis zu 5 Kohlenstoffatomen ist und $R^2$ ein Alkylrest gleich wie oder verschieden von $R^1$.

2. Methode zum Extrahieren von Astaxanthin gemäß Anspruch 1, dadurch gekennzeichnet, daß das Verhältnis von trockenen Muscheln zu Lösungsmittel zwischen 1:3 und 1:5 variiert.

3. Methode zum Extrahieren von Astaxanthin gemäß Anspruch 1, dadurch gekennzeichnet, daß der Vorgang innerhalb des Temperaturbereichs durchgeführt wird, worin das Lösungsmittel in flüssigem Zustand ist.

4. Methode zum Extrahieren von Astaxanthin wie im vorhergehenden Anspruch beansprucht, dadurch gekennzeichnet, daß der Arbeitsgang vorzugsweise bei einer Temperatur zwischen 15 und 25°C durchgeführt wird.

**Revendications**

1. Procédé d'extraction de l'astaxanthine des carapaces de crustacés consistant à traiter les carapaces avec un solvant choisi parmi les esters alkyliques d'acides carboxyliques ayant la formule générale

$$R^1{-}\overset{O}{\overset{\|}{C}}OR^2$$

dans laquelle $R^1$ est l'hydrogène ou un radical alkyl ayant jusqu'à 5 atomes de carbone, et $R^2$ est un radical alkyle identique à, ou différent de, $R^1$.

2. Procédé d'extraction de l'astaxanthine selon la revendication précédente, dans lequel le rapport des carapaces sèches au solvant varie entre 1:3 et 1:5.

3. Procédé d'extraction de l'astaxanthine selon la revendication 1, dans lequel l'opération est effectuée dans la plage de températures où le solvant est à l'état liquide.

4. Procédé d'extraction de l'astaxanthine selon la revendication précédente, dans lequel l'opération est effectuée de préférence à une température comprise entre 15 et 25°C.